# EUROPEAN PATENT SPECIFICATION

(11) **EP 3 962 270 B1**
(45) Date of publication and mention of the grant of the patent: **23.07.2025**
(21) Application number: 20720799.4
(22) Date of filing: 20.04.2020
(51) Int. Cl.: A01N 25/02, A01N 25/10, A01N 43/58, A01P 7/04

(54) **STABILIZATION OF SUSPENSION CONCENTRATES BY HYDROPHOBIC FUMED SILICA**
STABILISIERUNG VON SUSPENSIONSKONZENTRATEN DURCH HYDROPHOBE PYROGENE KIESELSÄURE
STABILISATION DE CONCENTRÉS EN SUSPENSION AU MOYEN DE LA SILICE SUBLIMÉE HYDROPHOBE

(30) Priority: 03.05.2019 US 201962842920 P
(43) Date of publication of application: 09.03.2022
(73) Proprietor: BASF SE, 67056 Ludwigshafen am Rhein (DE)
(72) Inventor: XU, Wen, Research Triangle Park, NC 27709 (US); FLOYD, Robert M, Research Triangle Park, NC 27709 (US); BENTON, Kara Walden, Research Triangle Park, NC 27709 (US)
(74) Representative: BASF IP Association
(86) International application number: PCT/EP2020/060983
(87) International publication number: WO 2020/224943

(56) References cited:
- EP-A1- 3 329 777
- WO-A1-2018/083040
- WO-A1-2020/224943
- US-A- 6 074 987
- US-A1- 2018 184 654

## Description

The invention relates to an aqueous agrochemical composition comprising suspended particles of dimpropyridaz that has a water-solubility of at least 1 g/l at 25 °C, and hydrophobically modified fumed silica. The invention also relates to a process for the preparation of the agrochemical composition; to a method of controlling phytopathogenic fungi and/or undesired plant growth and/or undesired insect or mite attack and/or for regulating the growth of plants, wherein the agrochemical composition is allowed to act on the respective pests, their environment or the crop plants to be protected from the respective pest, on the soil and/or on undesired plants and/or on the crop plants and/or on their environment. It also relates to the agrochemical composition for use in a method of controlling or preventing infestation by insects or parasites of an animal. Another object is the use of the hydrophobically modified fumed silica for stabilizing an aqueous agrochemical composition comprising the suspended particles of dimpropyridaz; and a method of stabilizing an aqueous agrochemical composition comprising the suspended particles of dimpropyridaz comprising the contacting of the hydrophobically modified fumed silica with particles of the active ingredient and water. Combinations of embodiments with other embodiments, regardless of their respective level of preference, are within the scope of the invention.

Aqueous suspension concentrates are one of the most commonly used formulation types in the agrochemical industry. They are usually confined to active ingredients that have a very low water-solubility. Active ingredients with a high water-solubility are difficult to build into an aqueous suspension concentrates, since the particles either dissolve during storage or undergo Ostwald-Ripening, thereby resulting in increased particle size and eventually sedimentation. Other stability problems of such formulations are gelling, i.e. the formation of unstructured conglomerates of the active ingredient negatively influence the desired rheological profiles, e.g. by increasing the viscosity, or by clogging spray nozzles.

On the other hand, aqueous suspension concentrates have the advantage of a low content of organic solvents, and a relatively safe handling and application for the applicant.

It was therefore desirable to supply an aqueous agrochemical composition that contains dimpropyridaz with high water-solubility in the form of suspended particles, which is stable upon storage, does not undergo particle growth, particle aggregation or conglomeration, and thus is not prone to gelling or sedimentation of particles.

The objective has been achieved by an aqueous agrochemical composition comprising
a) suspended particles of dimpropyridaz; and
b) hydrophobically fumed silica;
wherein the active ingredient has a water-solubility of at least 1 g/l at 25 °C.

It was surprisingly found that the hydrophobic modification of the fumed silica is crucial to generate the advantageous composition of the present invention.

The agrochemical composition is an aqueous agrochemical composition. The water content of the agrochemical composition is typically at least 1 wt%, preferably at least 5 wt%, more preferably at least 10 wt%, more preferably at least 15 wt%, most preferably at least 20 wt%, utmost preferably at least 25 wt%, especially preferably at least 30 wt%, and in particular at least 35 wt%, each time based on the total weight of the agrochemical compositions.

The water content of the agrochemical composition is typically up to 95 wt%, preferably up to 90 wt%, more preferably up to 80 wt%, most preferably up to 70 wt%, especially preferably up to 60 wt%, and in particular up to 50 wt% based on the total weight of the agrochemical composition. The water content of the agrochemical composition is typically of from 10 to 85 wt%, preferably from 10 to 65 wt%, more preferably from 15 to 60 wt% based on the total weight of the agrochemical composition.

The agrochemical composition comprises hydrophobically modified fumed silica, which is typically present in the form of suspended particles. Hydrophobically modified silica is commercially available, inter alia under the trade names of Aerosil, e.g. Aerosil R805.

The term "fumed silica" as used herein is synonymous with the term "pyrogenous silica". It refers to amorphous silica, which may for example be produced from a precursor, e.g. SiCl₄ by pyrolysis in a flame, or from quartz sand vaporized in an electric arc.

The term "hydrophobically modified" as used herein refers to the modification of the fumed silica, typically on the surface of the fumed silica particles. The modification usually results in the covalent attachment of at least one hydrocarbon chain to the fumed silica, preferably to the surface of the fumed silica particles. Typically, the term "hydrophobically modified fumed silica" refers to fumed silica having at least one covalently attached hydrocarbon chain containing of from 1 to 20 carbon atoms, preferably from 1 to 16, more preferably from 1 to 10 carbon atoms.

Hydrophobically modified fumed silica is typically not covalently attached to a hydrocarbon chain that is substituted with a hydroxyl group, a carboxylate group, a sulfonate group, or a phosphonate group.

The hydrophobically modified fumed silica is typically present in form of suspended particles in the aqueous agrochemical composition. The particles may be characterized by their size distribution, which can be determined by dynamic light scattering techniques. Suitable dynamic light scattering measurement units are inter alia produced under the trade name Malvern Mastersizer 3000. The particles may be characterized by their median diameter, which is usually abbreviated as x50 value. The x50 value refers to a particular particle diameter, wherein half of the particle population by volume is smaller than this diameter. The x50 value is typically determined according to ISO 13320:2009.

The particles may have an x50 value of from 0.5 nm to 1 µm, preferably from 1 nm to 500 nm, more preferably from 5 to 100 µm, most preferably from 10 µm to 50 µm. The particles typically have an x50 value of at least 0.75 nm, preferably at least 2nm.

Thy hydrophobically modified fumed silica is non-porous. It may have a BET-surface of from at least 50 m²/g, preferably at least 60 m²/g, more preferably at least 80 m²/g. The hydrophobically modified fumed silica may have a BET-surface of up to 400 m²/g, preferably up to 350 m²/g, more preferably up to 300 m²/g. The hydrophobically modified fumed silica may have a BET-surface of from 10 to 500 m²/g, preferably from 50 to 400 m²/g. The BET surface may be determined according to DIN ISO 9277:2014-01.

The concentration of the hydrophobically modified fumed silica may be at least 0.01 wt%, preferably at least 0.1 wt%, more preferably at least 0.2 wt%, most preferably at least 0.3 wt%, and in particular ate least 0.4 wt%, such as at least 0.5 wt% based on the total weight of the agrochemical composition. The concentration of the hydrophobically modified fumed silica may be up to 15 wt%, preferably up to 10 wt%, more preferably up to 5 wt%, most preferably up to 1.8 wt% based on the total weight of the agrochemical composition. The concentration of the hydrophobically modified fumed silica is typically from 0.1 to 10 wt%, preferably from 0.1 to 5 wt%, more preferably from 0.3 to 2 wt%, most preferably from 0.4 to 1.7 wt% based on the total weight of the agrochemical composition.

The weight ratio of the active ingredient to the hydrophobically modified fumed silica is typically from 1000:1 to 1:1, preferably from 500:1 to 5:1, more preferably from 200:1 to 20:1, most preferably from 100:1 to 25:1. Usually, the weight ratio of the active ingredient to the hydrophobically modified fumed silica is at least 15:1, preferably 18:1, more preferably 22:1.

Thy hydrophobically modified fumed silica typically has a carbon content of at least 0.1 wt%, preferably at least 0.5 wt% based on the total weight of the hydrophobically modified silica. The hydrophobically modified fumed silica may have a carbon content of up to 10 wt%, preferably up to 8 wt% based on the total weight of the hydrophobically modified silica.

The agrochemical composition comprises a pesticidally effective amount of the active ingredient. The term "effective amount" denotes an amount of the active ingredient, which is sufficient for controlling harmful fungi on cultivated plants or in the protection of materials and which does not result in a substantial damage to the treated plants. Such an amount can vary in a broad range and is dependent on various factors, such as the pest species to be controlled, the treated cultivated plant or material, the climatic conditions and the specific active ingredient used.

The concentration of the active ingredient in the agrochemical composition is typically at least 5 wt%, more preferably at least 10 wt%, most preferably at least 15 wt%, especially preferably at least 20 wt%, utmost preferably at least 25 wt%, and in particular at least 30 wt% based on the total weight of the agrochemical composition. The concentration of the active ingredient in the agrochemical composition is typically up to 95 wt%, preferably up to 85 wt%, more preferably up to 75 wt%, especially preferably up to 75 wt%, and in particular up to 65 wt% based on the total weight of the agrochemical composition. The agrochemical composition typically contains the active ingredient in a concentration of from 10 to 90 wt%, preferably of from 15 to 60 wt%, more preferably of from 20 to 50 wt% based on the total weight of the agrochemical composition.

The active ingredient is present in the agrochemical composition in the form of suspended particles. The particles may be characterized by their size distribution, which can be determined by dynamic light scattering techniques. Suitable dynamic light scattering measurement units are inter alia produced under the trade name Malvern Mastersizer 3000. The particles may be characterized by their median diameter, which is usually abbreviated as x50 value. The x50 value refers to a particular particle diameter, wherein half of the particle population by volume is smaller than this diameter. The x50 value is typically determined according to ISO 13320:2009.

The particles may have an x50 value of from 0.05 µm to 30 µm, preferably from 0.1 µm to 20 µm, more preferably from 0.5 to 20 µm, most preferably from 0.5 µm to15 µm, especially preferably from 0.5 µm to 10 µm. The particle typically have an x50 value of at least 0.75 µm, preferably at least 1 µm.

The active ingredient has a water-solubility of at least 1 g/l at 20 °C. Preferably, the active ingredient has a water-solubility of at least 5 g/l, preferably at least 10 g/l, more preferably at least 20 g/l, most preferably at least 30 g/l, and in particular at least 40 g/l. The active ingredient may have a water-solubility at 25 °C of up to 200 g/l, preferably up to 100 g/, more preferably up to 80 g/l, most preferably up to 60 g/l, utmost preferably up to 50 g/l. The active ingredient may have a water-solubility at 25 °C of from 15 g/l to 70 g/l, preferably from 25 g/l to 40 g/l.

The agrochemical composition may contain a further active ingredient, which may be selected from fungicides, insecticides, nematicides, herbicides, safeners, micronutrients, biopesticides, nitrification inhibitors, urease inhibitors, and/or growth regulators. The further active ingredient may be present in dissolved form or as suspended particles in the agrochemical composition. In one embodiment, the further active ingredient is present as an emulsified liquid.

The concentration of the further active ingredient is typically from 1 to 50 wt%, preferably from 10 to 25 wt% based on the total weight of the agrochemical composition.

The agrochemical composition may be prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005. The agrochemical composition is typically prepared by contacting the hydrophobically modified fumed silica with the active ingredient, preferably in the presence of water. In one embodiment, the resulting mixture is then subjected to milling or grinding to produce the suspension of the active ingredient. In another embodiment, the active ingredient is contacted with water and milled or grinded to produce an aqueous suspension of the active ingredient, whereupon said aqueous suspension is contacted with the hydrophobically modified fumed silica.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin. Suitable anti-foaming agents are silicones, long chain alcohols, and salts of fatty acids.

Suitable colorants (e.g. in red, blue, or green) are pigments of low water solubility and watersoluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

The agrochemical composition may optionally comprise further auxiliaries, such as 0,1-1 wt% bactericides, 5-15 wt% anti-freezing agents, 0,1-1 wt% anti-foaming agents, and 0,1-1 wt% colorants.

Solutions for seed treamtent (LS), Suspoemulsions (SE), flowable concentrates (FS), are usually employed for the purposes of treatment of plant propagation materials, particularly seeds. The compositions in question give, after two-to-tenfold dilution, active substance concentrations of from 0.01 to 60% by weight, preferably from 0.1 to 40% by weight, in the ready-to-use preparations. Application can be carried out before or during sowing. Methods for applying the agrochemical composition on to plant propagation material, especially seeds include dressing, coating, pelleting, dusting, soaking and in-furrow application methods of the propagation material. Preferably, the agrochemical composition are applied on to the plant propagation material by a method such that germination is not induced, e. g. by seed dressing, pelleting, coating and dusting.

When employed in plant protection, the amounts of active ingredient applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active ingredient of from 0.1 to 1000 g, preferably from 1 to 1000 g, more preferably from 1 to 100 g and most preferably from 5 to 100 g, per 100 kilogram of plant propagation material (preferably seeds) are generally required.

When used in the protection of materials or stored products, the amount of active ingredient applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners) may be added to the agrochemical composition as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank and further auxiliaries may be added, if appropriate.

### Application methods

The agrochemical composition is suitable for use in protecting crops, plants, plant propagation materials, such as seeds, or soil or water, in which the plants are growing, from attack or infestation by animal pests. Therefore, the present invention also relates to a plant protection method, which comprises contacting crops, plants, plant propagation materials, such as seeds, or soil or water, in which the plants are growing, to be protected from attack or infestation by animal pests, with a pesticidally effective amount of the agrochemical composition.

The agrochemical composition suitable for use in combating or controlling animal pests. Therefore, the present invention also relates to a non-therapeutic method of combating or controlling animal pests, which comprises contacting the animal pests, their habitat, breeding ground, or food supply, or the crops, plants, plant propagation materials, such as seeds, or soil, or the area, material or environment in which the animal pests are growing or may grow, with a pesticidally effective amount of the agrochemical composition of the present invention.

The agrochemical composition is effective through both contact and ingestion. Furthermore, the agrochemical composition can be applied to any and all developmental stages, such as egg, larva, pupa, and adult.

The application can be carried out both before and after the infestation of the crops, plants, plant propagation materials, such as seeds, soil, or the area, material or environment by the pests.

Suitable application methods include inter alia soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the agrochemical composition to the furrow, and closing the furrow. Foliar application refers to the application of the agrochemical composition to plant foliage, e.g. through spray equipment. For foliar applications, it can be advantageous to modify the behavior of the pests by use of pheromones in combination with agrochemical composition. Suitable pheromones for specific crops and pests are known to a skilled person and publicly available from databases of pheromones and semiochemicals, such as
http://www.pherobase.com.

As used herein, the term "contacting" includes both direct contact (applying the agrochemical composition directly on the animal pest or plant - typically to the foliage, stem or roots of the plant) and indirect contact (applying the agrochemical composition to the locus, i.e. habitat, breeding ground, plant, seed, soil, area, material or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests.

The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet or fodder beet; fruits, such as pomes, stone fruits or soft fruits, e.g. apples, pears, plums, peaches, nectarines, almonds, cherries, papayas, strawberries, raspberries, blackberries or gooseberries; leguminous plants, such as beans, lentils, peas, alfalfa or soybeans; oil plants, such as rapeseed (oilseed rape), turnip rape, mustard, olives, sunflowers, coconut, cocoa beans, castor oil plants, oil palms, ground nuts or soybeans; cucurbits, such as squashes, pumpkins, cucumber or melons; fiber plants, such as cotton, flax, hemp or jute; citrus fruit, such as oranges, lemons, grapefruits or mandarins; vegetables, such as eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, such as avocados, cinnamon or camphor; energy and raw material plants, such as corn, soybean, rapeseed, sugar cane or oil palm; tobacco; nuts, e.g. walnuts; pistachios; coffee; tea; bananas; vines (table grapes and grape juice grape vines); hop; sweet leaf (also called Stevia); natural rubber plants or ornamental and forestry plants, such as flowers (e.g. carnation, petunias, geranium/pelargoniums, pansies and impatiens), shrubs, broad-leaved trees (e.g. poplar) or evergreens, e.g. conifers; eucalyptus; turf; lawn; grass such as grass for animal feed or ornamental uses. Preferred plants include potatoes sugar beets, tobacco, wheat, rye, barley, oats, rice, corn, cotton, soybeans, rapeseed, legumes, sunflowers, coffee or sugar cane; fruits; vines; ornamentals; or vegetables, such as cucumbers, tomatoes, beans or squashes.

The term "cultivated plants" is to be understood as including plants which have been modified by mutagenesis or genetic engineering in order to provide a new trait to a plant or to modify an already present trait.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hectare, 30 to 40 g per hectare, or 40 to 50 g per hectare.

Advantages: the agrochemical composition is characterized by a very high storage stability, little particle growth, reduced sedimentation, reduced gelling, an advantageous rheological profile, and high biological efficacy. The term "stability" as used herein typically refers to physical stability of the agrochemical formulation. Accordingly, the term "stabilizing" usually refers to a physical stabilization (e.g. increase of storage stability

The following examples illustrate the invention.

### Examples

The following ingredients were used for preparing the agrochemical compositions of the examples.
Insecticide A: 1-[(1*RS*)-1,2-dimethylpropyl]*-N-*ethyl-5-methyl*-N-*pyridazin-4-yl-1*H-*pyrazole-4-carboxamide.
Dispersant: lignosulfonate, organic sulfur content approximately 9 wt%
Antifoam: emulsion of dimethylsiloxan on silica particles, defoamer content 20 wt%
Biocide A: glycol based solution of benzisothiazolinone
Biocide B: aqueous composition of benzisothiazolinones and 5-chloro-2-methylisothiazolin-3-one
Silica Particles A: hydrophilic fumed silica particles, BET surface 200 m²/g, mean particle diameter 12 nm.
Silica Particles B: hydrophilic fumed silica particles, BET surface 300 m²/g.
Silica Particles C: hydrophilic fumed silica particles, BET surface 50 m²/g.
Silica Particles D: hydrophobic fumed silica particles, BET surface 220-280 m²/g, surface modified with octamethylcyclotetrasiloxane, carbon content 1.5-3 wt%.
Silica Particles E: hydrophobic fumed silica particles, BET surface 125-175 m²/g, surface modified with octylsilyl groups, carbon content 4.5-6.5 wt%.
Silica Particles F: surface treated fumed silica particles, hydrophobic and hydrophilic properties, BET surface 190 m²/g, produced by surface modification of Silica Particles B with hexa-decylsilane, carbon content 0.9-1.8 wt%.
Silica Particles G: hydrophobic fumed silica particles, surface modified with dimethyldichlorosilane, water-solubility below 0.1 g/l, BET surface 90-130 m²/g, carbon content 0.6 to 1.2 wt%
Silica Particles H: hydrophobic fumed silica particles, BET surface 150-190 m²/g, surface modified with dimethyldichlorosilane, carbon content 0.8-1.4 wt%.
Wet A: ethoxylated castor oil, 40 polymerized ethylene oxide units per molecule on average, saponification value 58-66 (according to ISO 3657).

### Example-1:

Suspension concentrates SC-1 to SC-8, and control SC-C1 were prepared containing the ingredients according to Table A. The suspension concentrates differed by the type of Silica Particle used, as provided in Table B, wherein SC-C1 did was produced without any Silica Particles.

The suspension concentrates were prepared by adding in a first step Insecticide A, 50% of the total amount of the Wet A, Dispersing Agent, Antifoam, Biocide A, Biocide B, acetic acid, and, in case of SC-1 to SC-8, the Silica Particles to water.

The resulting mill base was homogenized in a second step until uniform, and then milled in a bead mill until a mean particle size of 2-3 microns was achieved. The resulting composition was mixed in a third step with xanthan gum, which had been hydrated as a 3 wt% dilution in water, and the remaining Wet A until homogenous.

The suspension concentrates were then placed in a 20 °C / 40 °C cycling chamber for one day or one month (24 hours at 20 °C and then 24 hours at 40 °C), upon which the suspension concentrates were analyzed by visual inspection and by testing the rheological properties. The results of these analyses were summarized in Table B.

**Table A: Ingredients of suspension concentrates SC-1 to SC-8**

| **Component** | **Concentration [wt%]** |
|---|---|
| Insecticide A | 20.85 |
| Wet A | 8.00 |
| Dispersant | 2.00 |
| Antifoam | 0.40 |
| Biocide A | 0.20 |
| Biocide B | 0.10 |
| Xanthan guml | 0.13 |
| Acetic acid | 0.09 |
| Silica Particles | 1.00 |
| Water | to 100 |

**Table B: Silica Particles contained in suspension concentrates SC-1 to SC-8; and results of stability analysis after incubation in the cycling chamber. SC-C1 did not contain any Silica Particles, but bas balanced with water.**

| Suspension Concentrate | Silica Particles | Stability Analyses after incubation in the cycling chamber | |
|---|---|---|---|
| | | Incubation one day | Incubation one month |
| SC-C1 | Balanced by water | Gelled | Gelled |
| SC-1 | Silica Particles A | Gelled | Gelled |
| SC-2 | Silica Particles B | Gelled | Gelled |
| SC-3 | Silica Particles C | Gelled | Gelled |
| SC-4 | Silica Particles D | Flowable | Flowable |
| SC-5 | Silica Particles E | Flowable | Flowable |
| SC-6 | Silica Particles F | Flowable | Slightly Flowable |
| SC-7 | Silica Particles G | Flowable | Flowable |
| SC-8 | Silica Particles H | Flowable | Flowable |

### Example-2:

Suspension concentrates SC-9 to SC-13, and SC-C2 were prepared containing the ingredients according to Table C. The suspension concentrates differed by the concentration of Silica Particles G, as provided in Table D.

The suspension concentrates were prepared by adding in a first step Insecticide A, 50% of the total amount of the Wet A, Dispersing Agent, Antifoam, Biocide A, Biocide B, acetic acid, and the allotted amount of the Silica Particles G to water.

The resulting mill base was homogenized in a second step until uniform, and then milled in a bead mill until a mean particle size of 2-3 microns was achieved. The resulting composition was mixed in a third step with xanthan gum, which had been hydrated as a 3 wt% dilution in water, and the remaining Wet A until homogenous.

The suspension concentrates were then placed in a 20 °C / 40 °C cycling chamber for two weeks (24 hours at 20 °C and then 24 hours at 40 °C), upon which the suspension concentrates were analyzed by visual inspection and by testing the rheological properties. The results of these analyses were summarized in Table D.

**Table C: Ingredients of suspension concentrates SC-9 to SC-14**

| **Component** | **Concentration [wt%]** |
|---|---|
| Insecticide A | 31.82 |
| Wet A | 8.00 |
| Dispersant | 3.00 |
| Antifoam | 0.40 |
| Biocide A | 0.20 |
| Biocide B | 0.10 |
| Xanthan guml | 0.06 |
| Acetic acid | 0.15 |
| Silica Particles G | see Table D |
| Water | to 100 |

**Table D: Silica Particles contained in suspension concentrates SC-9 to SC-13; and results of stability analysis after incubation in the cycling chamber. SC-C2 did not contain any Silica Particles.**

| Suspension Concentrate | Concentration of Silica Particles G in wt% | Stability Analyses after incubation in the cycling chamber |
|---|---|---|
| SC-C2 | 0.0 | Gelled |
| SC-9 | 0.5 | Flowable |
| SC-10 | 1.0 | Flowable |
| SC-11 | 1.5 | Flowable |
| SC-12 | 2.0 | Slightly Flowable |
| SC-13 | 3.0 | Slightly Flowable |

## Claims

1. Aqueous agrochemical composition comprising
a) suspended particles of dimpropyridaz; and
b) hydrophobically modified fumed silica,
where said agrochemical composition comprises dimpropyridaz at a concentration of at least 10 wt% based on the total weight of the agrochemical composition.

2. Agrochemical composition of claim 1, containing the silica in a concentration of from 0.01 to 10 wt% based on the total weight of the agrochemical composition.

3. Agrochemical composition according to any of claims 1 to 2, containing the silica in a concentration of at least 0.1 wt% based on the total weight of the agrochemical composition.

4. Agrochemical composition according to any of claims 1 to 3 having a weight ratio of the dimpropyridaz to the silica from 500:1 to 5:1.

5. Agrochemical composition according to any of claims 1 to 4, wherein the silica has a BET-surface of from 10 to 500 m²/g according to DIN ISO 9277:2014-01.

6. Agrochemical composition according to any of claims 1 to 5, wherein the silica has a carbon content of at least 0.5 wt% based on the total weight of the hydrophobically modified silica.

7. The agrochemical composition according to any of claims 1 to 6, wherein the suspended particles of the active ingredient have a mean diameter of from 0.5 µm to 20 µm.

8. The agrochemical composition according to any of claims 1 to 7, wherein the hydrophobically fumed silica has at least one covalently attached hydrocarbon chain containing from 1 to 20 carbon atoms.

9. The agrochemical composition according to any of claims 1 to 8, containing a second active ingredient selected from insecticides, fungicides, and herbicides.

10. Use of hydrophobically modified fumed silica as defined in any of claims 1 to 8 for stabilizing an aqueous agrochemical composition comprising suspended particles of dimpropyridaz as defined in any of claims 1 to 9.

11. Method of stabilizing an aqueous agrochemical composition comprising the suspended particles of dimpropyridaz as defined in any of claims 1 to 9 comprising the step of contacting the hydrophobically modified fumed silica as defined in any of claims 1 to 8 with particles of dimpropyridaz as defined in any of claims 1 to 9 and water.

12. Composition as defined in any of claims 1 to 9 for use in a method of controlling or preventing infestation by insects or parasites of an animal.

13. Method of preparing the agrochemical composition according to any of claims 1 to 9 comprising the step of contacting the hydrophobically modified fumed silica as defined in any of claims 1 to 8 with dimpropyridaz as defined in any of claims 1 to 9 in the presence of water.

14. Plant propagation material containing the agrochemical composition as defined in any of claims 1 to 9.

15. Non-therapeutic Use of the agrochemical composition according to any of claims 1 to 9 for combating or controlling animal pests.

## Patentansprüche

1. Wässrige agrochemische Zusammensetzung, umfassend
a) suspendierte Partikel von Dimpropyridaz; und
b) hydrophob modifizierte pyrogene Kieselsäure,
umfassend die agrochemische Zusammensetzung Dimpropyridaz in einer Konzentration von mindestens 10 Gew.-%, bezogen auf das Gesamtgewicht der agrochemischen Zusammensetzung.

2. Agrochemische Zusammensetzung nach Anspruch 1, die die Kieselsäure in einer Konzentration von 0,01 bis 10 Gew.-%, bezogen auf das Gesamtgewicht der agrochemischen Zusammensetzung, enthält.

3. Agrochemische Zusammensetzung nach einem der Ansprüche 1 bis 2, die die Kieselsäure in einer Konzentration von mindestens 0,1 Gew.-%, bezogen auf das Gesamtgewicht der agrochemischen Zusammensetzung, enthält.

4. Agrochemische Zusammensetzung nach einem der Ansprüche 1 bis 3, mit einem Gewichtsverhältnis von Dimpropyridaz zu Siliciumdioxid von 500:1 bis 5:1.

5. Agrochemische Zusammensetzung nach einem der Ansprüche 1 bis 4, wobei die Kieselsäure eine BET-Oberfläche von 10 bis 500 m2/g gemäß DIN ISO 9277:2014-01 aufweist.

6. Agrochemische Zusammensetzung nach einem der Ansprüche 1 bis 5, wobei die Kieselsäure einen Kohlenstoffgehalt von mindestens 0,5 Gew.-%, bezogen auf das Gesamtgewicht der hydrophob modifizierten Kieselsäure, aufweist.

7. Agrochemische Zusammensetzung nach einem der Ansprüche 1 bis 6, wobei die suspendierten Partikel des Wirkstoffs einen mittleren Durchmesser von 0,5 µm bis 20 µm aufweisen.

8. Die agrochemische Zusammensetzung gemäß einem der Ansprüche 1 bis 7, wobei die hydrophob modifizierte pyrogene Kieselsäure mindestens eine kovalent gebundene Kohlenwasserstoffkette mit 1 bis 20 Kohlenstoffatomen aufweist.

9. Die agrochemische Zusammensetzung gemäß einem der Ansprüche 1 bis 8, die einen zweiten Wirkstoff enthält, der aus Insektiziden, Fungiziden und Herbiziden ausgewählt ist.

10. Verwendung von hydrophob modifiziertem pyrogenem Siliciumdioxid gemäß einem der Ansprüche 1 bis 8 zur Stabilisierung einer wässrigen agrochemischen Zusammensetzung, die suspendierte Partikel von Dimpropyridaz gemäß einem der Ansprüche 1 bis 9 umfasst.

11. Verfahren zur Stabilisierung einer wässrigen agrochemischen Zusammensetzung, die suspendierte Partikel von Dimpropyridaz gemäß einem der Ansprüche 1 bis 9 umfasst, umfassend den Schritt des Inkontaktbringens der hydrophob modifizierten pyrogenen Kieselsäure gemäß einem der Ansprüche 1 bis 8 mit Partikeln von Dimpropyridaz gemäß einem der Ansprüche 1 bis 9 und Wasser.

12. Zusammensetzung gemäß einem der Ansprüche 1 bis 9 zur Verwendung in einem Verfahren zur Bekämpfung oder Verhinderung des Befalls eines Tieres durch Insekten oder Parasiten.

13. Verfahren zur Herstellung der agrochemischen Zusammensetzung gemäß einem der Ansprüche 1 bis 9, umfassend den Schritt des Inkontaktbringens der hydrophob modifizierten pyrogenen Kieselsäure gemäß einem der Ansprüche 1 bis 8 mit Dimpropyridaz gemäß einem der Ansprüche 1 bis 9 in Gegenwart von Wasser.

14. Pflanzenvermehrungsmaterial, das die in einem der Ansprüche 1 bis 9 definierte agrochemische Zusammensetzung enthält.

15. Nicht-therapeutische Verwendung der agrochemischen Zusammensetzung gemäß einem der Ansprüche 1 bis 9 zur Bekämpfung oder Kontrolle von tierischen Schädlingen.

## Revendications

1. Composition agrochimique aqueuse comprenant
a) des particules en suspension de dimpropyridaz ; et
b) de la silice fumée modifiée de manière hydrophobe,
ladite composition agrochimique comprenant du dimpropyridaz à une concentration d'au moins 10 % en poids par rapport au poids total de la composition agrochimique.

2. Composition agrochimique selon la revendication 1, contenant la silice à une concentration comprise entre 0,01 et 10 % en poids par rapport au poids total de la composition agrochimique.

3. Composition agrochimique selon l'une quelconque des revendications 1 à 2, contenant la silice à une concentration d'au moins 0,1 % en poids par rapport au poids total de la composition agrochimique.

4. Composition agrochimique selon l'une quelconque des revendications 1 à 3, ayant un rapport pondéral du dimpropyridaz à la silice compris entre 500:1 et 5:1.

5. Composition agrochimique selon l'une quelconque des revendications 1 à 4, dans laquelle la silice a une surface BET comprise entre 10 et 500 m2/g selon la norme DIN ISO 9277:2014-01.

6. Composition agrochimique selon l'une quelconque des revendications 1 à 5, dans laquelle la silice a une teneur en carbone d'au moins 0,5 % en poids par rapport au poids total de la silice modifiée de manière hydrophobe.

7. Composition agrochimique selon l'une quelconque des revendications 1 à 6, dans laquelle les particules en suspension de l'ingrédient actif ont un diamètre moyen compris entre 0,5 µm et 20 µm.

8. Composition agrochimique selon l'une quelconque des revendications 1 à 7, dans laquelle la silice fumée hydrophobe comporte au moins une chaîne hydrocarbonée liée de manière covalente contenant de 1 à 20 atomes de carbone.

9. Composition agrochimique selon l'une quelconque des revendications 1 à 8, contenant un deuxième ingrédient actif choisi parmi les insecticides, les fongicides et les herbicides.

10. Utilisation de silice pyrogénée modifiée de manière hydrophobe telle que définie dans l'une quelconque des revendications 1 à 8 pour stabiliser une composition agrochimique aqueuse comprenant des particules en suspension de dimpropyridaz telles que définies dans l'une quelconque des revendications 1 à 9.

11. Procédé de stabilisation d'une composition agrochimique aqueuse comprenant les particules en suspension de dimpropyridaz telles que définies dans l'une quelconque des revendications 1 à 9, comprenant l'étape consistant à mettre en contact la silice pyrogénée modifiée de manière hydrophobe telle que définie dans l'une quelconque des revendications 1 à 8 avec des particules de dimpropyridaz telles que définies dans l'une quelconque des revendications 1 à 9 et de l'eau.

12. Composition selon l'une quelconque des revendications 1 à 9, destinée à être utilisée dans un procédé de contrôle ou de prévention de l'infestation par des insectes ou des parasites d'un animal.

13. Procédé de préparation de la composition agrochimique selon l'une quelconque des revendications 1 à 9, comprenant l'étape consistant à mettre en contact la silice fumée modifiée de manière hydrophobe telle que définie dans l'une quelconque des revendications 1 à 8 avec le dimpropyridaz tel que défini dans l'une quelconque des revendications 1 à 9 en présence d'eau.

14. Matériel de multiplication végétale contenant la composition agrochimique telle que définie dans l'une quelconque des revendications 1 à 9.

15. Utilisation non thérapeutique de la composition agrochimique selon l'une quelconque des revendications 1 à 9 pour lutter contre ou contrôler les animaux nuisibles.
